(19) Europäisches Patentamt
European Patent Office
Office européen des brevets

(11) Veröffentlichungsnummer **0 007 519**
**B1**

(12) **EUROPÄISCHE PATENTSCHRIFT**

(45) Veröffentlichungstag der Patentschrift: **04.11.81**

(51) Int. Cl.³: **C 07 C 143/822**

(21) Anmeldenummer: **79102375.7**

(22) Anmeldetag: **11.07.79**

(54) **Verfahren zur Herstellung von Hydroxyalkyl-perfluoralkan-sulfonamiden.**

(30) Priorität: **22.07.78 DE 2832346**

(43) Veröffentlichungstag der Anmeldung: **06.02.80 Patentblatt 80/3**

(45) Bekanntmachung des Hinweises auf die Patenterteilung: **04.11.81 Patentblatt 81/44**

(84) Benannte Vertragsstaaten: **BE DE FR GB IT NL**

(56) Entgegenhaltungen:
**AT-B-242 118**
**DE-A-2 307 377**
**DE-B-1 140 188**
**DE-B2-2 024 909**
**DE-C-738 703**
**GB-A-1 298 291**
**US-A-2 803 656**
**LIEBIGS ANNALEN DER CHEMIE, Bd. 731, 1970, Weinheim**
**V. BEYL, H. NIEDERPRÜM und P. VOSS »Eine neue Reaktion von Perfluoralkylsulfonylfluoriden« Seiten 58 — 66**

(73) Patentinhaber: **BAYER AG, Zentralbereich Patente, Marken und Lizenzen, D-5090 Leverkusen 1, Bayerwerk (DE)**

(72) Erfinder: **Mitschke, Karl-Heinz, Dr., Am Berg 22, D-5068 Odenthal (DE)**
Erfinder: **Geisler, Klaus, Dr., Petrusstrasse 7, D-5300 Bonn-Beuel (DE)**
Erfinder: **Niederprüm, Hans, Dr., Hofstrasse 27, D-4019 Monheim (DE)**

**EP 0 007 519 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

Verfahren zur Herstellung von Hydroxyalkyl-perfluoralkan-sulfonamiden

Die vorliegende Erfindung betrifft ein neues Verfahren zur Herstellung von Hydroxyalkyl-perfluoralkansulfonamiden der allgemeinen Formeln

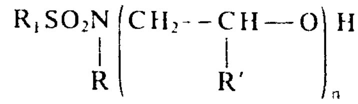

bzw.

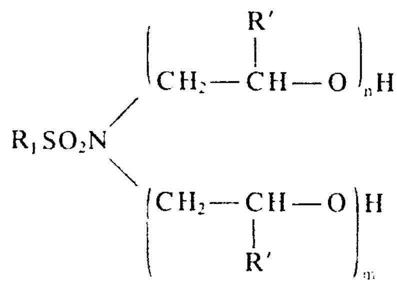

wobei

$R_F$ für einen geradlinigen oder verzweigten Perfluoralkylrest mit 1 – 20 C-Atomen, vorzugsweise 1 – 10 C-Atomen

R und R' unabhängig voneinander für Wasserstoff, einen Alkyl-, Hydroxyalkyl-, Cycloalkyl-, Aralkyl-, Alkenyl- oder Arylrest, wobei R' auch für Halogenalkyl stehen kann, und

n und m unabhängig voneinander für 1 – 30 stehen.

Hydroxyalkyl-perfluoralkan-sulfonamide sind vielseitig einsetzbar, insbesondere als Zwischenprodukte zur Herstellung von Oleophobierungs- oder Hydrophobierungsmittel für die Imprägnierung von Textilien, Leder, Papier und ähnlichen Stoffen bzw. als hochoberflächenaktive Mittel. Ferner finden diese Produkte Verwendung als Verlaufsmittel für Wachse, Fette, Lacke und andere Stoffe.

Die Herstellung von Hydroxyalkyl-perfluoralkan-sulfonamiden erfolgte bisher (vgl. z. B. US-Patentschrift 2 803 656) durch Umsetzung der Alkalisalze der Perfluoralkansulfonsäureamide mit Halogenhydrinen.

Dieses Verfahren ist jedoch sehr aufwendig, da es sich um eine Zweistufenreaktion handelt. Zunächst werden die Sulfonamide in alkoholischer Lösung mit einem Alkalihydroxid in das entsprechende Salz überführt. Nach Abdestillieren des Lösungsmittels wird dann die eigentliche Reaktion mit dem Chlor- oder Bromalkanol durchgeführt. Zum anderen handelt es sich um ein heterogenes Reaktionssystem, es sind also lange Reaktionszeiten (in der Größenordnung von mehr als 10 Std.) notwendig. Hierbei treten jedoch schon erhebliche Zersetzungsreaktionen auf, so daß eine umständliche Reinigung notwendig wird und die Ausbeuten vermindert werden.

Die an sich naheliegende Umsetzung zur Herstellung von Perfluoralkyl-sulfonamidoalkanolen, die Reaktion von Perfluoralkyl-sulfonylfluorid mit z. B. N-Alkyläthanolamin, ist nicht durchführbar, da nicht nur die NH- sondern auch die OH-Gruppe mit dem Sulfonylhalogenid reagiert und der gebildete Ester wegen seiner stark alkylierenden Eigenschaften eine Reine von Folgereaktionen eingeht. Auch eine Blockierung der OH-Funktion, z. B. durch Silylierung, führt nicht zum Ziel, da die Trimethylsilylgruppe sehr rasch eine Austauschreaktion mit dem Sulfonylfluorid unter Bildung von Trimethylfluorsilan eingeht und höher alkylierte Stickstoffverbindungen entstehen (vgl. Ann. 731, 58 [1970]).

Einem weiteren Verfahren, gemäß DE-OS 2 307 377, liegt die Umsetzung von Perfluoralkylsulfonamiden mit Epoxiden zwar zugrunde, jedoch sind als Katalysatoren nur tert. Amine geeignet. Alkalihydroxide, -Alkoholate und andere Katalysatoren weitgehend versagen. Zusätzlich ist es notwendig, in Lösungsmitteln zu arbeiten, was die Aufarbeitung der Reaktionsprodukte aufwendig macht.

Aufgabe der vorliegenden Erfindung war es nun, ein einfaches und wirtschaftliches Verfahren zur Herstellung von Hydroxylalkylperfluoralkansulfonamiden bereitzustellen.

Gegenstand der vorliegenden Erfindung ist ein Verfahren zur Herstellung von Hydroxyalkylperfluoralkansulfonamiden der allgemeinen Formeln

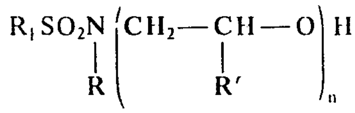

bzw.

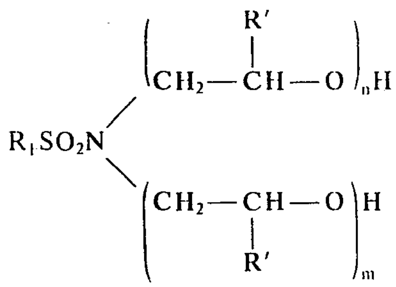

wobei

$R_F$ für einen geradlinigen oder verzweigten Perfluoralkylrest mit 1—20 Atomen,
R und R' unabhängig voneinander für Wasserstoff, einen Alkyl-, Hydroxyalkyl-, Cycloalkyl-, Aralkyl-, Alkenyl- oder Arylrest, wobei R' auch für Halogenalkyl stehen kann, und
m und n unabhängig voneinander für einen Wert von 1—30 stehen,

dadurch gekennzeichnet, daß Perfluoralkylsulfonamide der allgemeinen Formel

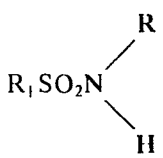

mit Epoxiden der allgemeinen Formel

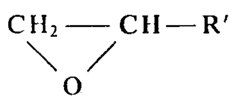

wobei $R_F$, R und R' die obengenannte Bedeutung besitzen, in der Schmelze bei Temperaturen von 40 bis 170°C, in Gegenwart von basisch reagierenden organischen und anorganischen Metallverbindungen und/oder basischen Ionenaustauschern umgesetzt werden.

R und R' sind vorzugsweise Wasserstoff, Alkylreste mit 1—6 C-Atomen, die gegebenenfalls mit Hydroxy oder im Falle von R' auch mit Halogen substituiert sein können, Cycloalkylreste mit 3 bis 8 C-Atomen, Benzyl, Phenetyl, Alkenyl mit bis zu 4 C-Atomen, Phenyl oder Naphthyl.

Überraschend hat es sich herausgestellt, daß die erfindungsgemäße Umsetzung, entgegen der Lehre der DE-OS 2 307 377, z. B. mit Alkalihydroxiden oder -Alkoholaten glatt gelingt, und daß auch die Verwendung von Lösungsmitteln nicht notwendig ist. Auf diese Weise wird die Aufarbeitung sehr erleichtert, denn eine Reinigung der anfallenden Produkte, etwa durch Vakuumdestillation, ist nicht mehr erforderlich. Technisch ist nämlich diese Reinigungsmethode bei derartigen Substanzen äußerst umständlich und nachteilig, da trotz der angewandten niedrigen Drucke von weniger als 1,33 mbar teilweise immer noch Sumpftemperaturen bis 200°C erforderlich sind, was zwangsläufig zu einem hohen Zersetzungsgrad führt.

Das erfindungsgemäße Verfahren wird in der Weise durchgeführt, daß in die Schmelze eines Perfluoralkylsulfonamides mit einem entsprechenden Katalysator die Epoxide eingetragen werden. Die Umsetzungen erfolgen bei Temperaturen von 40—170°C, vorzugsweise zwischen 60 und 140°C. Die Umsetzungen können vorzugsweise bei Normal- jedoch auch bei erhöhtem Druck (beispielsweise im Autoklaven) durchgeführt werden. Weiterhin ist es möglich, diskontinuierlich oder kontinuierlich zu arbeiten.

Bei Verwendung von basischen Ionenaustauschern können diese nach der Reaktion, beispielsweise durch Filtration der Schmelze, abgetrennt und gegebenenfalls wiederverwendet werden. Beim Einsatz von beispielsweise Alkalihydroxiden oder ähnlichen Substanzen können die Reaktionsprodukte durch Waschen mit heißem Wasser, gegebenenfalls unter Druck, und anschließender Filtration und Trocknung im Vakuum von den zugesetzten Katalysatoren gereinigt werden. Jedoch können die

Katalysatoren auch im Produkt verbleiben.

Als Ausgangsmaterialien werden Perfluoralkylsulfonamide verwendet, die bekanntlich durch Umsetzung der entsprechenden Fluoride mit Ammoniak, primären oder sekundären Aminen erhältlich sind. Die Sulfonamide können gegebenenfalls destilliert werden. Wenn eine Reinigung durch Destillation erfolgen soll, ist es bei dieser Stufe aufgrund des günstigeren Molekulargewichtes vorteilhafter als beim Endprodukt.

Beispiele für die zu verwendenden Perfluoralkylsulfonamide sind u. a.:

$CF_3SO_2NH-CH_3$, $CF_3SO_2NH_2$, $C_4F_9SO_2NH_2$, $C_4F_9SO_2NH-CH_3$,

$C_4F_9SO_2NH-C_4H_9$, $C_4F_9SO_2NH-CH_2-CH{=}CH_2$

$C_8F_{17}SO_2NH_2$, $C_8F_{17}SO_2NH-CH_3$, $C_8F_{17}SO_2NH-C_5H_{11}$,

$C_8F_{17}SO_2NH-CH_2C_6H_5$.

Als Alkylenoxide kommen prinzipiell alle Epoxidgruppen enthaltenden Verbindungen in Frage wie beispielsweise:

Äthylenoxid, Propylenoxid, Glycidol, Styroloxide, Butylenoxide, Epichlorhydrin, Phenoxypropylenoxide oder Gemische untereinander, insbesondere jedoch Epoxide mit 2–4 C-Atomen.

Als Katalysatoren sind basisch wirkende organische oder anorganische Metallverbindungen oder auch basische Ionenaustauscher geeignet.

Unter basisch wirkende anorganische Metallverbindungen fallen beispielsweise Alkali-Oxide, -Hydroxide, -Carbonate oder -Phosphate wie etwa NaOH, KOH, $K_2CO_2$ oder $Na_3PO_4$ oder Erdalkali-Oxide oder -Hydroxide oder Alkali- oder Erdalkali-Hydride wie etwa NaH.

Unter basisch wirkende organische Metallverbindungen fallen Metall-Alkyle oder -Aryle wie Buthyllithium oder Phenylnatrium, oder sek. Metallamide wie Lithium-diisopropylamid oder Metallalkoholate wie Natriummethylat oder Kalium-tert.-butylat oder Metallcarboxylate wie Natriumacetat oder den Alkalisalzen der einzusetzenden Sulfonamide $R_FSO_2N(R)Na$.

Die Katalysatoren werden in Mengen von 0,01–20 Mol-%, vorzugsweise von 0,2–10 Mol-%, bezogen auf das entsprechende Sulfonamid eingesetzt.

Als basische Katalysatoren eignen sich auch Ionenaustauscher, deren Träger beispielsweise aus Polystyrol oder Formaldehyd-Phenol-Harzen bestehen und deren für den Ionenaustausch charakteristische Gruppen quartäre Ammoniumsalze sind. Solche Stoffe sind beispielsweise die von der BAYER AG vertriebenen Lewatite®, die wie starke Basen wirken. Die Mengen liegen bei 0,1–20 g pro Mol eingesetztes Sulfonamid.

Ein größerer Anteil an Katalysatoren ist grundsätzlich möglich, wirtschaftlich jedoch nicht sinnvoll. Ebensowenig sinnvoll ist es, die Katalysatormenge herabzusetzen.

Das erfindungsgemäße Verfahren soll nun anhand einiger Beispiele näher erläutert werden.

## Beispiel 1

In einem Dreihalskolben, versehen mit Rührer, Innenthermometer, Trockeneis-Rückflußkühler und kühlbarem Tropftrichter, an dessen Auslauf ein bis auf den Boden des Kolbens reichendes Einleitungsrohr angebracht ist, wurden bei ca. 120°C zu einer Schmelze von 256,6 g (0,5 Mol) N-Methyl-perfluoroctansulfonamid und 0,5 g (0,0089 Mol) Kaliumhydroxid innerhalb von 30 Min. 24,2 g (0,55 Mol) Ethylenoxid zugetropft und anschließend ca. 2,5 Stunden nachgerührt. Nach dem Entfernen von unverbrauchtem Ethylenoxid (bzw. Epoxide allgem.) oder anderer flüchtiger Verbindungen im Vakuum wurden 279 g (~quant. incl. Katalys.) des Produktes der Formel

$$C_8F_{17}SO_2N(CH_3)CH_2CH_2OH$$

vom Festbereich 90–95°C und der OH-Zahl von 3,1% (theor. 3,05%) erhalten, dessen Zusammensetzung NMR-spektroskopisch und gaschromatographisch bestimmt wurden.

Um den Katalysator zu entfernen, wurde das Produkt mit heißem Wasser gewaschen, die Mischung unter Rühren abgekühlt, wobei das Produkt körnig anfiel, filtriert und im Vakuum getrocknet. Festbereich 94–97°C.

## Beispiel 2

Analog Beispiel 1 wurden aus 499 g (1 Mol) Perfluoroctansulfonamid, 2 g (0,036 Mol) Kaliumhydroxid und 92,5 g (2,1 Mol) Ethylenoxid bei ca. 120°C innerhalb von 4 Std. 592 g (~quant. incl. Katalys.) der Verbindung

$$C_8F_{17}SO_2N(CH_2-CH_2-OH)_2$$

vom Festbereich 136—140° C und der OH-Zahl von 5,77% (theor 5,79%) erhalten, die NMR-spektroskopisch und gaschromatographisch identifiziert wurde.

Beispiel 3

Analog Beispiel 1 wurden aus 802,5 g (12,66 Mol) Perfluorbutansulfonamid, 2 g (0,036 Mol) Kaliumhydroxid und 248 g (5,63 Mol) Ethylenonid bei 90—100° C innerhalb von 4 Stunden 1039 g (~quant. incl. Katalys.) vom Produkt der Formel

$$C_4F_9SO_2N(CH_2-CH_2-OH)_2$$

mit einem Schmelzbereich um 75° C und der OH-Zahl von 8,78% (theor. 8,78%) erhalten, die NMR-spektroskopisch und gaschromatographisch identifiziert wurde.

Beispiel 4

Entsprechend der Versuchsanordnung aus Beispiel 1 wurden 156,6 g (0,5 Mol) N-Methyl-perfluorbutansulfonamid und 24,2 g (0,55 Mol) Ethylenoxid in Gegenwart von 10 g basischem Ionenaustauscher MP 500 (Hydroxyl-Ionen tragende Gruppen an vernetztem Polystyrol) bei 70—90° C innerhalb von ca. 2 Stunden umgesetzt. Nach dem Abfiltrieren des Ionenaustauschers und Entfernen des überschüssigen Ethylenoxids im Vakuum wurden 178 g (~quant.) Produkt der Formel

$$C_4F_9SO_2N(CH_3)CH_2CH_2OH$$

erhalten.

Beispiel 5

Entsprechend der Versuchsanordnung aus Beispiel 1 wurden aus 78,3 g (0,25 Mol) N-Methyl-perfluorbutansulfonamid, 1 g (0,009 Mol) Kalium-tert.-butylat und 15,3 g (0,263 Mol) Propylenoxid bei einer Temperatur von 100—120° C innerhalb von ca. 2,5 Stunden und nach dem Entfernen des überschüssigen Propylenoxids im Vakuum 94 g (~quant. incl. Katalysator) der Verbindung

$$C_4F_9SO_2N(CH_3)CH_2-CH(CH_3)OH$$

mit einem Schmelzbereich von 38—41° C. Nach der Wäsche mit heißem Wasser, analog Beispiel 1, lag der Festbereich bei 45—48° C.

Beispiel 6

Aus 78,3 g (0,25 Mol N-Methylperfluorbutansulfonamid, entsprechend Beispiel 1, 1,2 g (0,0087 Mol) Kaliumcarbonat und 15,3 g (0,263 Mol) Propylenoxid erhielt man 94 g (~quant. incl. Katalys.) der Verbindung

$$C_4F_9SO_2N(CH_3)-CH_2-CH(CH_3)OH$$

vom Festbereich 38—40° C, die NMR-spektroskopisch und gaschromatographisch identifiziert wurde. Nach der Wäsche mit heißem Wasser lag der Festbereich bei 45—48° C.

Beispiel 7

Aus 81,6 g (0,5 Mol) N-Methyl-perfluormethansulfonamid, 1 g (0,018 Mol) Kaliumhydroxid und 32,5 g (0,55 Mol) Propylenoxid erhielt man, analog Beispiel 1, 112 g (~quant. incl. Katalys.) der Verbindung

$$CF_3SO_2N(CH_3)CH_2-CH(CH_3)OH$$

die NMR-spektroskopisch identifiziert wurde.

Beispiel 8

Entsprechend erhielt man aus 49,9 g (0,1 Mol) Perfluoroctansulfonamid, dem 10 ml Dimethylformamid zugesetzt wurden, 200 mg (0,0036 Mol) Kaliumhydroxid und 16,5 g (0,22 Mol) Glycidol bei 120 – 130° C 65 g ( ~ quant. incl. Katalys.) der Verbindung

$$C_8F_{17}\text{-}SO_2N(CH_2—CH(OH)—CH_2OH)_2$$

die NMR-spektroskopsich bestätigt wurde.

Beispiel 9

Entsprechend der Versuchsanordnung aus Beispiel 1 wurden 78,3 g (0,25 Mol) N-Methyl-perfluorbutansulfonamid, 0,3 g (0,0075 Mol) Natriumhydroxid und 19 g (0,26 Mol) Ethyloxiran bei ca. 120 C innerhalb von 3 Std. 97 g ( ~ quant. incl. Katalys.) des Produktes

$$C_4F_9SO_2N(CH_3)CH_2—CH(OH)C_2H_5$$

erhalten, das NMR-spektroskopisch und gaschromatographisch identifiziert wurde.

Beispiel 10

Analog wurden aus 78,3 g (0,25 Mol) N-Methylperfluorbutansulfonamid, 0,5 g (0,0089 Mol) Kaliumhydroxid und 30,7 g (0,26 Mol) [(2-Propylenoxy)methyl]oxiran bei ca. 110 C innerhalb von 3 Stunden 108,8 g ( ~ quant. incl. Katalys.) der Verbindung

$$C_4F_9SO_2N(CH_3)CH_2—CH(OH)—CH_2—O—CH_2—CH{=}CH_2$$

erhalten, das NMR-spektroskopisch und gaschromatographisch identifiziert wurde.

Beispiel 11

Entsprechend wurden aus 78,3 g (0,25 Mol) N-Methylperfluorbutansulfonamid, 0,5 g (0,0089 Mol) Kaliumhydroxid und 39,3 g (0,26 Mol) Phenoxymethyloxiran bei 120—130° C innerhalb von 3 Std. 117 g ( ~ quant. incl. Katalys.) der Verbindung

$$C_4F_9SO_2N(CH_3)CH_2—CH(OH)—CH_2—O—C_6H_5$$

vom Festbereich 62—65° C erhalten, die NMR-spektroskopisch und gaschromatographisch identifiziert wurde.

Beispiel 12

Entsprechend wurden aus 31,3 g (0,1 Mol) N-Methylperfluorbutansulfonamid, 0,1 g (0,0018 Mol) Kaliumhydroxid und 7,4 g (0,1 Mol) Glycidol bei ca. 120° C innerhalb von 3 Std. 38,7 g ( ~ quant. incl. Katalys.) der Verbindung

$$C_4F_9SO_2N(CH_3)CH_2—CH(OH)—CH_2OH$$

vom Festpunkt um 95° C erhalten, die NMR-spektroskopisch und gaschromatographisch identifiziert wurde.

Beispiel 13

Entsprechend wurden aus 31,3 g (0,1 Mol) N-Methyl-perfluorbutansulfonamid, 0,1 g (0,0025 Mol) Natriumhydroxid und 14,8 g (0,2 Mol) Glycidol bei 120—130° C innerhalb von 4 Stunden 46 g ( ~ quant. incl. Katalys.) der Verbindung

$$C_4F_9SO_2N(CH_3)CH_2—CH(OH)—CH_2—O—CH_2—CH(OH)—CH_2—OH$$

vom Festpunkt 69° C erhalten, die NMR-spektroskopisch und gaschromatographisch identifiziert wurde.

Beispiel 14

Entsprechend Beispiel 1 wurden zu 47 g (0,15 Mol) N-Methylperfluorbutansulfonamid und 0,5 g (0,0089 Mol) Kaliumhydroxid bei 100—120° C 44 g (1 Mol) Ethylenoxid innerhalb von 7 Std. zugetropft. Nach dem Entfernen von überschüssigem Ethylenoxid im Vakuum erhielt man 87 g einer gut wasserlöslichen Flüssigkeit der mittleren Zusammensetzung

$$C_4F_9SO_2N(CH_3)(CH_2—CH_2—O)_6H$$

Beispiel 15

In einem Autoklaven wurden 626 g (2 Mol) N-Methylperfluorbutansulfonamid und 5 g (0,089 Mol) Kaliumhydroxid eingebracht, die Mischung bei ca. 90° C aufgeschmolzen und die Apparatur mit Stickstoff unter einen Druck von ca. 5 bar gesetzt. Mittels einer Lewa-Pumpe wurden 122 g (2,1 Mol) Propylenoxid zudosiert. Nach ca. 1,5 Stunden wurde die Apparatur entspannt, überschüssiges Propylenoxid im Vakuum entfernt und die Schmelze abgesaugt. Man erhielt die Verbindung analog Beispiel 5, die NMR-spektroskopisch und gaschromatographisch identifiziert wurde.

Beispiel 16

In einer kleinen Autoklavapparatur wurden 27,7 g (0,054 Mol) N-Methylperfluoroctansulfonamid, 0,2 g (0,005 Mol) Natriumhydroxid und 66 g (1,5 Mol) Ethylenoxid eingebracht und die Mischung unter Rühren auf 120—130° C erhitzt, wobei sich ein Druck von ca. 10 bar einstellte.

Als nur noch ein Druck von ca. 2 bar gemessen wurde, wurde die Apparatur über eine Kühlfalle entspannt und danach im Vakuum entgast. Man erhielt 78 g einer wachsartigen, gut wasserlöslichen Substanz der mittleren Zusammensetzung

$$C_8F_{17}SO_2N(CH_3)(CH_2—CH_2—O)_{22}H$$

Beispiel 17

Analog der Versuchsanordnung aus Beispiel 1 wurden aus 313 g (1 Mol) N-Methyl-perfluorbutansulfonamid, 0,6 g (0,015 Mol) Natriumhydroxid und 48,5 g (1,1 Mol) Ethylenoxid bei 80—90° C innerhalb von 3 Stunden 357 g (~quant. incl. Katalysator) der Verbindung

$$C_4F_9SO_2N(CH_3)CH_2CH_2OH$$

vom Festpunkt 61—63° C erhalten, die NMR-spektroskopisch und gaschromatographisch identifiziert wurde.

Beispiel 18

Entsprechend der Versuchsanordnung aus Beispiel 1 erhielt man aus 76,3 g (0,2 Mol) N-Cyclohexylperfluorbutansulfonamid, 0,96 g (0,009 Mol) Lithium-diisopropylamid und 12,8 g (0,22 Mol) Propylenoxid 88 g (~quant. incl. Katalys.) der Verbindung

$$C_4F_9SO_2N(C_6H_{11})CH_2—\underset{\displaystyle CH_3}{\underset{|}{CH}}—OH$$

Beispiel 19

Entsprechend der Versuchsanordnung aus Beispiel 1 wurden aus 77,9 g (0,2 Mol) N-Benzyl-perfluorbutansulfonamid, 0,01 Mol (gelöst in Hexan) Butyllithium und 10 g (0,23 Mol) Ethylenoxid 87 g (~quant. incl. Katalys.) der Verbindung

$C_4F_9SO_2N(CH_2-C_2H_5)CH_2-CH_2-OH$

erhalten.

Beispiel 20

Entsprechend erhielt man aus 78,3 g (0,25 Mol) N-Methylperfluorbutansulfonamid 3 g (0,0085 Mol) vom Kaliumsalz des Sulfonamides und 15,25 g (0,26 Mol) Propylenoxid 96 g (~quant. incl. Katalys.) der Verbindung

$C_4F_9SO_2N(CH_3)CH_2CH(CH_3)OH$

**Patentansprüche**

1. Verfahren zur Herstellung von Hydroxyalkylperfluor-alkansulfonamiden der allgemeinen Formeln

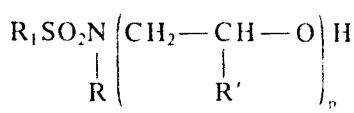

bzw.

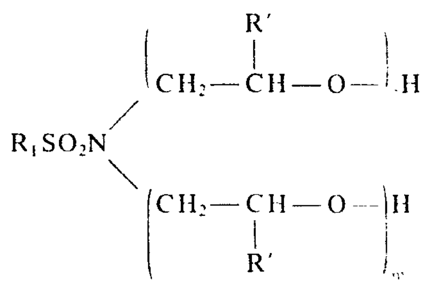

wobei

$R_F$ für einen geradlinigen oder verzweigten Perfluoralkylrest mit 1—20 C-Atomen,
R und R' unabhängig voneinander für Wasserstoff, einen Alkyl-, Hydroxyalkyl-, Cycloalkyl-, Aralkyl-, Alkenyl- oder Arylrest, wobei R' auch für Halogenalkyl stehen kann, und
m und n unabhängig voneinander für einen Wert von 1—30 stehen,

dadurch gekennzeichnet, daß Perfluoralkylsulfonamide der allgemeinen Formel

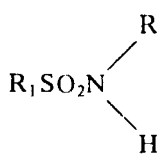

mit Epoxiden der allgemeinen Formel

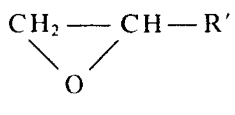

wobei $R_F$, R und R' die obengenannte Bedeutung besitzen, in der Schmelze bei Temperaturen von 40 bis 170°C, in Gegenwart von basisch reagierenden organischen und anorganischen Metallverbindungen und/oder basischen Ionenaustauschern umgesetzt werden.

2. Verfahren nach Anspruch 1, dadurch gekennzeichnet, daß als Katalysator Kaliumhydroxid und/oder Natriumhydroxid eingesetzt wird.

**Claims**

1. Process for the preparation of hydroxyalkyl perfluoroalkane sulphonamides of the general formulae

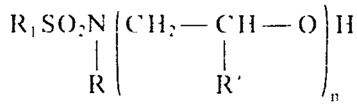

or

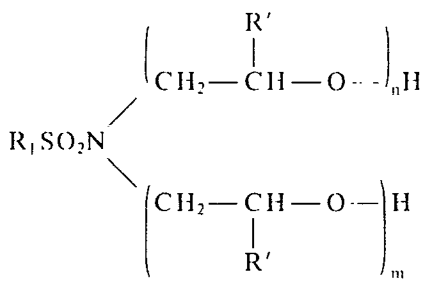

wherein

$R_f$ represents a straight- oder branched-chain perfluoroalkyl grop having 1—20 C atoms,
R and R′ each independently represents hydrogen or an alkyl, hydroxyalkyl, cycloalkyl, aralkyl, alkenyl or aryl group, is being possible for R′ to also represent halogenoalkyl, and
m and n each independently represents a value of from 1—30,

characterized in that perfluoroalkyl sulphonamides of the general formula

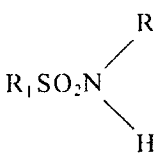

are reacted with epoxides of the general formula

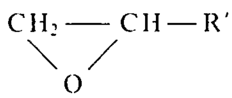

in which $R_f$, R and R′ have the abovementioned meaning, without solvents at temperatures of from 40 to 170°C, in the presence of organic and inorganic metal compounds which are basic in reaction and/or basic ion exchangers.

2. Process according to Claim 1, characterized in that potassium hydroxide and/or sodium hydroxide is used as the catalyst.

**Revendications**

1. Procédé de préparation d'hydroxyalkyl-perfluoralcane-sulfonamides répondant à une des formules générales suivantes:

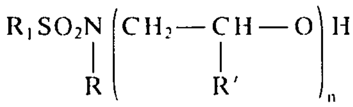

ou

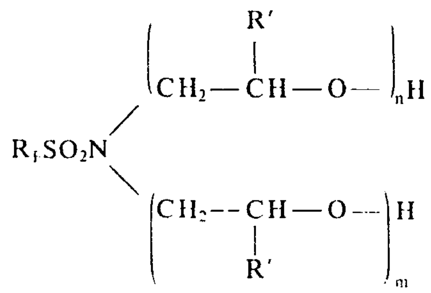

où

| | |
|---|---|
| $R_F$ | représente un groupe perfluoralkyle à chaîne droite ou ramifiée contenant 1 à 20 atomes de carbone, |
| R et R' | représentent chacun indépendamment l'un de l'autre un atome d'hydrogène, un groupe alkyle, un groupe hydroxyalkyle, un groupe cycloalkyle, un groupe aralkyle, un groupe alcényle ou un groupe aryle, R' pouvant également représenter un groupe halogénalkyle, et |
| m et n | ont, indépendamment l'un de l'autre, une valeur de 1—30, |

caractérisé en ce qu'on fait réagir des perfluoralkyl-sulfonamides de formule générale:

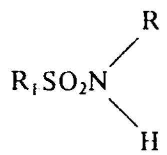

avec des époxydes de formule générale:

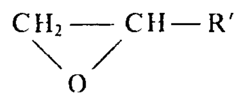

où $R_F$, R et R' ont les significations indiquées cidessus, en masse fondue, à des températures de 40 à 170°C, en présence de composés métalliques organiques et inorganiques à réaction basique et/ou d'échangeurs d'ions basiques.

2. Procédé suivant la revendication 1, caractérisé en ce que, comme catalyseur, on utilise l'hydroxyde de potassium et/ou l'hydroxyde de sodium.